# EUROPÄISCHE PATENTSCHRIFT

(11) **EP 0 743 949 B1**
(45) Veröffentlichungstag und Bekanntmachung des Hinweises auf die Patenterteilung: **15.12.1999**
(21) Anmeldenummer: 95908258.7
(22) Anmeldetag: 03.02.1995
(51) Int. Cl.: C07H 1/08, C12N 15/10, C12P 19/34

(54) **VERFAHREN ZUR HERSTELLUNG ENDOTOXINFREIER ODER AN ENDOTOXIN ABGEREICHERTER NUCLEINSÄUREN UND/ODER OLIGONUCLEOTIDE FÜR DIE GENTHERAPIE**
PROCESS FOR PRODUCING ENDOTOXIN-FREE OR ENDOTOXIN-POOR NUCLEIC ACIDS AND/OR OLIGONUCLEOTIDES FOR GENE THERAPY
PROCEDE DE PREPARATION D'ACIDES NUCLEIQUES ET/OU D'OLIGONUCLEOTIDES SANS ENDOTOXINES OU PAUVRES EN ENDOTOXINES UTILES EN THERAPIE GENIQUE

(30) Priorität: 07.02.1994 DE 4403692; 25.06.1994 DE 4422291; 01.09.1994 DE 4431125; 14.09.1994 DE 4432654
(43) Veröffentlichungstag der Anmeldung: 27.11.1996
(73) Patentinhaber: QIAGEN GmbH, 40724 Hilden (DE)
(72) Erfinder: COLPAN, Metin, D-45219 Essen (DE); SCHORR, Joachim, D-40231 Düsseldorf (DE); MORITZ, Peter, D-50169 Kerpen (DE)
(74) Vertreter: Meyers, Hans-Wilhelm, Dr.Dipl.-Chem.
(86) Internationale Anmeldenummer: EP9500389
(87) Internationale Veröffentlichungsnummer: WO9521177

(56) Entgegenhaltungen:
- WO-A-93/11221
- US-A- 4 997 932
- DATABASE WPI Section Ch, Week 8310 Derwent Publications Ltd., London, GB; Class A96, AN 83-23220 & JP-A-58 013 519 (SEIKAGAKU KOGYO KK) , 26.Januar 1983

## Beschreibung

Gegenstand der vorliegenden Erfindung ist ein Verfahren zur Isolierung und Reinigung von Nucleinsäuren und/oder Oligonucleotiden für den Einsatz in der Gentherapie, wobei die Nucleinsäuren und/oder Oligonucleotide aus einer im wesentlichen biologischen Quelle gereinigt werden, die Verwendung von Anionenaustauschermaterialien für die Trennung, Reinigung und Isolierung von Nucleinsäuren zur Herstellung eines nucleinsäurehaltigen Mittels für die Gentherapie sowie eine Zusammenstellung enthaltend Komponenten für die Durchführung des erfindungsgemäßen Verfahrens.

Eine neue Form der Therapie von genetisch bedingten Erkrankungen, wie cystische Fibrose oder Muskeldystrophie, basiert auf der Erkenntnis, daß diese Erkrankungen durch bestimmte Gendefekte verursacht werden. Wird dem erkrankten Organismus das gesunde Gen in ausreichendem Maße zur Verfügung gestellt, so scheint eine Therapie des Gendefektes möglich. Die Gentherapie ermöglicht nicht nur die Behandlung von genetisch bedingten Erkrankungen, sondern eignet sich auch zur Behandlung von Tumoren und ist als neue Form der Impfung gegen Infektionserkrankungen, wie Hepatitis, Influenza und HIV, um nur einige Beispiele zu nennen, geeignet (TIBTECH, Special Issue: Gene Therapy Therapeutic Strategy and Commercial Prospects, May 1993, Vol. 11, Nr. 5 (112)).

Ein zentrales Problem der Gentherapie besteht darin, die therapeutische DNA so zu verabreichen, daß diese den Ort des Geschehens erreicht. Bisher wurde den Patienten ein Teil der zu behandelnden Zellen, in denen das defekte Gen exprimiert wird, wie beispielsweise Blutzellen, entnommen. Diese Zellen wurden in Kulturschalen gezüchtet (in vitro). Um die fremde, therapeutisch wirkende DNA in die Zellen einzuschleusen, verwendete man z. B. Gensegmente eines Retrovirus, die mit der einzuschleusenden DNA verbunden wurden. Die genetisch veränderten Zellen wurden wieder in den Organismus verbracht (Anderson W. F. (1992), Human gene therapy, Science 256: 808 - 813).

Gegenwärtig werden bereits einige klinische Studien mit diesem sogenannten ex vivo Ansatz durchgeführt. Dabei verwendet man neuerdings neben den oben beschriebenen Retroviren auch Plasmid-DNA, Oligonucleotide, mRNA, genomische DNA, YACS (Yeast Artificial Chromosomes) für die Transfektion von Zellkulturen. Das ex vivo-Verfahren ist aber sehr arbeitsaufwendig und nicht zur Behandlung aller Erkrankungen geeignet. Als Beispiele seien die Muskeldystrophie oder die cystische Fibrose genannt. Es ist mithin wünschenswert, über einfachere Verfahrensweisen zu verfügen, um einem Organismus therapeutisch einsetzbare DNA zu applizieren. Dabei wurde gefunden, daß es möglich ist, Plasmid-DNA direkt in das Gewebe eines Organs zu applizieren. Ein Teil der DNA wird bis zum Zellkern transportiert. Die über die DNA verabreichte genetische Information wird dort in das therapeutisch wirkende Protein translatiert. Die Behandlung im Organismus erfolgt direkt und wird als in vivo-Behandlung bezeichnet.

Für die in vivo Behandlung kann man die DNA oder RNA auch mit Liposomen oder anderen Substanzen vermischen, was zu einer besseren Aufnahme der Nucleinsäuren in die Zelle führt. Die Nucleinsäure kann aber auch direkt in das zu behandelnde Organ, beispielsweise einen Muskel oder einen Tumor, injiziert werden (Plautz, G. E. et al. 1993, PNAS, Vol. 90, 4645 - 4649). Vorteilhaft dabei ist, daß die in den Organismus gelangende DNA keinerlei immunologische Reaktionen im Organismus hervorruft, sofern sie frei von immunogenen Begleitkontaminationen ist. Daher stellt die in vivo Gentherapie sehr hohe Anforderungen an die Qualität der zu applizierenden Nucleinsäuren. Die DNA muß frei von toxischen Substanzen sein, die zu pathogenen Effekten in den zu behandelnden Organismus führen können.

Klinische Phase-I-Studien am Menschen mit dieser Technologie haben zu sehr detaillierten und strengen Qualitätsanforderungen des für die hierbei verwendeten Nucleinsäuren geführt. Gemäß den Anforderungen des FDA in den USA müssen die für therapeutische Zwecke eingesetzten Nucleinsäuren die folgenden Qualitätskontrollen erfüllen:

| **Prüfung der Nucleinsäure auf:** | **Anforderung/Grenzwert** |
|---|---|
| Endotoxine | < 300 E.U./mg DNA |
| E.coli Genomische DNA | < 50 µg/mg DNA |
| Protein | < 100 µg/mg DNA |
| Supercoiled DNA | > 90% |
| A_{260/280} | 1,75 - 1,85 |
| Salzrückstände | Scan von A₂₂₀ - A₃₂₀ |
| RNA | < 1% |
| Sterilität | keine Kolonien nach 14 Tagen Tryptose Kultur |

Neben der Qualität der aufgereinigten Nucleinsäure, ist auch der Maßstab, in dem die Nucleinsäure aufgereinigt werden kann, von entscheidender Bedeutung. So muß es eine zukünftige Technologie ermöglichen, Nucleinsäuren im Maßstab von 1 mg bis zu 100 kg aufzureinigen, was wiederum Kulturvolumina von 1 1 bis zu 100 m³ erfordert.

Ein generelles Problem beim Aufreinigen von Nucleinsäuren aus Bakterienkulturen ist zunächst die Lyse der Mikroorganismen. Hierzu kann neben der von Birnborn und Dohly (Nucl. Acids Res. 7, Seiten 1513 - 1522 (1979)) beschriebenen und hier bevorzugten alkalischen Lyse auch das Aufbrechen der Bakterienzellen mittels hohem Druck (French Press), die Lyse in Anwesenheit von Detergenzien oder die Verwendung von Hitze (Boiling Lyse) angewendet werden.

Anschließend kann die Nucleinsäure durch unterschiedliche Verfahren mehr oder weniger effektiv von den übrigen Bestandteilen der Bakterienzelle, wie Proteinen oder genomischer DNA sowie Metaboliten abgetrennt werden. Als einfachste aber auch nicht sehr effiziente Möglichkeit, bietet sich die Abtrennung über die Zugabe von Salzen, wie z. B. LiCl an, was die Präzipitation der zellulären Proteine bewirkt. Die Nucleinsäure kann anschließend mit Alkohol präzipitiert werden. Ein Nachteil dieser Methode besteht darin, daß Kontaminationen von RNA, ssDNA und Proteinen nicht quantitativ abgetrennt werden können. Als zusätzlicher Reinigungsschritt wird oft noch eine Phenolextraktion durchgeführt, um vorhandene Proteinkontaminationen zu entfernen. Der Nachteil dieser als "Aussalzen" beschriebenen Methode besteht darin, daß vorhandene Endotoxin-Kontaminationen sowie RNA und ssDNA nicht beseitigt werden können. Die Phenolextraktion birgt zusätzlich die Gefahr der Verunreinigung der Nucleinsäure mit Phenol. Weiterhin führt eine Phenolbehandlung von Nucleinsäuren regelmäßig zu einem erhöhten Anteil von sogenannter "nicked" Nucleinsäure, d. h. Bruch des Nucleinsäurestranges an vielen Stellen, was wiederum dessen Stabilität stark beeinflußt.

Die CsCl Gradienten-Zentrifugation ist seit fast 30 Jahren eine etablierte Methode zur Reinigung von Nucleinsäuren.

Hierbei wird das unterschiedliche Sedimentationsverhalten von unterschiedlich großen Nucleinsäuremolekülen (RNA, Plasmid-DNA, genomische DNA) in einem CsCl Konzentrations-Gradienten in Anwesenheit von interkalierenden Agenzien, wie Ethidiumbromid, zur Separation von Nucleinsäuren genutzt. Diese Auftrennung kann nur bei großen Mengen eingesetzt werden. Dabei werden Ultrazentrifugen benötigt. Neben dem hohen materiellen Aufwand von ca. DM 60.000,-- pro Ultrazentrifuge wirkt sich der beträchtliche Zeitaufwand von mindestens 48 h für eine solche Aufreinigung als nachteilig aus. Dieses Verfahren ermöglicht nur eine Ausbeute von höchstens 5 mg Nucleinsäure pro Zentrifugenlauf.

An sich bekannt ist auch die Aufreinigung von Nucleinsäuren über chromatographische Verfahren. Hierbei sind generell zwei Verfahren zu unterscheiden.

Die Reinigung mit Hilfe der Anionenaustauscher-Chromatographie, wie sie in EP 0 268 946 B1 beschrieben ist. Hierbei werden die Bakterienzellen vorzugsweise über eine alkalische Lyse aufgeschlossen. Die zellulären Proteine und genomische DNA werden mit Hilfe von Detergenzien und anschließender Zentrifugation abgetrennt. Der so gewonnene, die Plasmid-DNA enthaltende Überstand, wird als Cleared Lysate bezeichnet. Das Cleared Lysate wird über eine Anionenaustauscher-Säule (Qiagen®) aufgereinigt, wobei eine quantitative Abtrennung von RNA und ssDNA erfolgt. Eine Entfernung von Endotoxinen findet hierbei nicht statt.

Gillespie und Vogelstein, Proc. Natl. Acad. Sci., USA, 76, S. 615 - 619, wurde beschrieben, daß eine Aufreinigung von Nucleinsäuren durch die Bindung an Silicagel oder Diatomeenerde in Anwesenheit von chaotropen Salzen, wie GuHCL, Nal usw. erfolgen kann. Im Gegensatz zur Anionenaustauscher-Chromatographie erfolgt die Bindung der DNA in Anwesenheit von hohen Salzkonzentrationen, wogegen die Elution bei niedrigen Salzkonzentrationen erfolgt. Der genaue Mechanismus ist nicht verstanden, jedoch wird von einer Präzipitation der Nucleinsäure auf der Oberfläche der Silicapartikel durch Dehydration, ausgegangen. Da hierbei die Bindung und Elution nach einem "alles oder nichts"-Prinzip stattfindet, ist keine quantitative Separation von RNA, ssDNA und Proteinen möglich. Daher sind diese DNA-Präparationen leider durch RNA, Protein und ssDNA-Kontaminationen für die Nucleinsäuregewinnung zum Einsatz in der Gentherapie nicht geeignet. Weiterhin finden sich 1000-fach höhere Endotoxinwerte in solchen Präparationen.

Die erhältlichen Nucleinsäuren sollten auch für den Einsatz in der Gentherapie nach der "Anti-sense"- oder "Sense"-Strategie geeignet sein. Die "Anti-sense"-Strategie nutzt die Eigenschaft von zum Beispiel mRNA mit komplementären Nucleinsäuren Hybride zu formen. Die Hybride sind inaktiv. Die "Anti-sense"-Nucleinsäure inaktiviert also die mRNA. Die erfindungsgemäß erhaltene "Anti-sense"-RNA kann dabei von außen kontinuierlich dem zu therapierenden Individuum zugeführt werden, oder von entsprechend transformierten Zellen im Individuum selbst gebildet werden. Die "Sense"-Strategie führt zur Ergänzung oder Unterstützung von z.B. mRNA, die wichtige Funktionen erfüllt. Dabei wird die benötigte RNA dem zu therapierenden Individuum zugeführt. Die nach erhältliche Nucleinsäure sollte auch geeignet sein, in sogenannten genetischen Vakzinierungsverfahren eingesetzt zu werden.

Die oben angeführten Qualitätsanforderungen können durch die beschriebenen DNA-Präparationsmethoden mit Hilfe der Caesiumchloridgradienten-Zentrifugation oder durch die Isolierung von DNA in Anwesenheit chaotroper Salze allein nicht befriedigt werden, da bei dieser Methode die zu isolierende DNA mit verschiedenen toxischen oder cancerogenen Substanzen, wie Phenol, Chloroform, Guanidiniumhydrochlorid, oder Ethidiumbromid in Berührung kommt. So läßt sich durch elektronenmikroskopische Analyse zeigen, daß in die Doppelhelix eingebautes Ethidiumbromid nicht mehr vollständig zu beseitigen ist (Schleef und Heinemann (Bio Techniques Vol. 14, Nr. 4, 1993). DNA-Moleküle, die während der Präparation, z. B. mit Ethidiumbromid kontaminiert werden, können im Körper aufgrund des interkalierten Ethidiumbromids allergische Reaktionen induzieren, wodurch jeder therapeutische Ansatz mit solcherart hergestellter DNA nicht zu vertreten ist.

Hochreine DNA kann ohne Verwendung toxischer Substanzen mit der Anionenaustauscher-Chromatographie präpariert werden. Allerdings können auch bei Anwendung der Chromatographie Endotoxine in nicht unbedenklichem Maße in die Nucleinsäuren und/oder Oligonucleotidfraktionen verschleppt werden.

Das der Erfindung zugrundeliegende technische Problem besteht in der Bereitstellung eines einstufigen Verfahrens zur Reinigung, Isolierung und Herstellung von Nucleinsäuren, die die hohen Qualitätsanforderungen an Nucleinsäuren und/oder Oligonucleotide für die Gentherapie erfüllen können. Dabei sollte nach Möglichkeit bereits bei der Probenvorbereitung eine drastische Endotoxinmengen-Verringerung erfolgen.

Überraschenderweise wird das der Erfindung zugrundeliegende technische Problem gelöst durch ein Verfahren, in dem das in Anspruch 1 genannte Anionenaustauscher-Chromatographiematerial wie folgt eingesetzt wird.

In der oben beschriebenen Weise kann durch unterschiedliche Methoden ein Cleared Lysate gewonnen werden. In einer bevorzugten Ausführung kann dabei auf den Zentrifugationsschritt nach der Lyse, zum Abtrennen der genomischen DNA und des SDS/Proteinkomplex durch die Verwendung einer in PCT/EP 95/00392 beschriebenen Filtrationseinrichtung verzichtet werden. Gleichzeitig ermöglicht diese Filtration eine erhebliche Reduktion der Endotoxin-Kontaminationen der Nucleinsäurelösung.

Die Nucleinsäure kann durch die Verwendung des in WO 95/21179 vorgeschlagenen Puffers in Verbindung mit der Anionenaustauscher-Chromatographie, Gelfiltration oder der Bindung an Silicagel, Diatomeenerde in Anwesenheit von chaotropen Salzen in einem einstufigen Prozeß so aufgereinigt werden, daß diese allen oben aufgeführten Qualitätsanforderungen genügt.

Das beim erfindungsgemäßen Verfahren eingesetzte Anionenaustauscher-Material ermöglicht aufgrund der um mindestens 100 mM NaCl auseinanderliegenden Elutionspunkte eine saubere Trennung von RNA und DNA.

Das im erfindungsgemäßen Verfahren verwendbare Anionenaustauschermaterial ist auch für die Aufreinigung von Viruspartikeln, insbesondere auch intakten Viruspartikeln, für die in vivo/ex vivo Gentherapie geeignet.

Endotoxine können jedoch auch wie in der WO 95/21179 vorgeschlagenen Verfahrensweise abgereichert oder entfernt werden. Dabei werden Endotoxine durch Behandlung mit chromatographischem Material abgereichert oder entfernt. Nach dem Aufschluß der natürlichen Quellen, aus denen die Nucleinsäuren und/oder Oligonucleotide gewonnen werden sollen, werden die erhaltenen Fraktionen metallchelatchromatographischen Methoden behandelt. Diese Methode ist neben der oder in Kombination mit der Inkubation der erhaltenen Fraktionen mit wäßrigen Salzlösungen und Detergenzien einsetzbar, wobei nach der Detergenz-Behandlung eine Anionenaustauscher-Chromatographie erfolgt. Die metallchelatchromatographischen Materialien umfassen Chelatbildner, IDA (Iminodiacetat) oder NTA (Nitrilotetraacetat), welche an Trägern, wie Silicagel, Diatomeenerde, Glas, Aluminiumoxide, Titanoxide, Zirkonoxide, Hydroxylapatit, Dextran, Agarose, Acrylamid, Polystyrol harze oder Copolymere aus den monomeren Baustein der genannten Polymere gebunden sind. Als weitere Materialien können auch Polymyxin oder DNA ETO® verwendet werden. Auf diesem Affinitätsträger können beispielsweise Nickelionen komplex gebunden werden, welche über weitere Koordinationsstellen mit in der Seitenkette stickstoffhaltigen Aminosäureresten in Proteinen in Wechselwirkung treten können. Die aufgeschlossenen, von Zelltrümmern befreiten biologischen Quellen können insbesondere mit Ni-NTA-Chromatographie-Material auf Kieselgelbasis inkubiert werden. Das Chromatographie-Material kann beispielsweise nach der erfolgten Inkubation abzentrifugiert werden, sofern im Batch-Verfahren gearbeitet wurde und der Überstand kann dann erfindungsgemäß weiter aufgearbeitet werden. Neben dem Batch-Verfahren kann aber auch die Affinitätschromatographie in Säulen durchgeführt werden, sofern die Beschaffenheit der Probe dies ermöglicht.

Die zu isolierende Nucleinsäure kann direkt aus Zellen, die lytisch aufgeschlossen werden, stammen. Das erfindungsgemäße Verfahren gewährleistet die Abtrennung von Kontaminanten und führt zu Nucleinsäuren, die die für die Gentherapie geforderte Reinheit aufweisen. Überraschenderweise zeigt insbesondere das im Handel befindliche Material QIAGEN® der Firma Qiagen seine Eignung im erfindungsgemäßen Verfahren eingesetzt zu werden.

Dieses Material ermöglicht eine sehr effiziente Abtrennung der DNA von RNA. Die DNA eluiert bei einer Salzkonzentration, die ca. 480 mM Natriumchlorid entspricht, wohingegen die doppelsträngige Plasmid-DNA erst bei ca. 1.260 mM Natriumchlorid eluiert. Die Differenz dieser beiden Elutionspunkte beträgt bei dem QIAGEN®-Material ca. 420 mM, wohingegen bei allen bekannten Anionenaustauscher-Materialien die Differenz zwischen den Elutionspunkten von RNA und Plasmid-DNA maximal ca. 80 mM Natriumchlorid-Konzentrationen beträgt. Eine derartig niedrige Differenz der Elutionspunkte beinhaltet eine hohe Gefahr der Coelution von DNA mit RNA, insbesondere einzelsträngiger DNA.

Das unter der Bezeichnung QIAGEN® im Handel erhältliche Material ist für die Reinigung von Plasmid-DNA für die Gentherapie besonders geeignet. Dieses chromatographische Trägermaterial ist ein modifiziertes poröses anorganisches Material. Als anorganische Trägermaterialien kommen Stoffe wie Silicagel, Diatomeenerde, Glas, Aluminiumoxide, Titanoxide, Zirkonoxide, Hydroxylapatit und als organische Trägermaterialien solche wie Dextran, Agarose, Acrylamid, Polystyrolharze oder Copolymere aus den monomeren Bausteinen der genannten Polymere in Frage.

Der Anionenaustauscher, der vorzugsweise eingesetzt wird, ist beispielsweise durch die Umsetzung eines der oben genannten Trägermaterialien in einem ersten Schritt mit einem Silanisierungsreagenz der allgemeinen Formel I erhältlich,

R¹R²R³Si R⁴ (I),

wobei
R¹ ein Alkoxyrest mit 1 bis 10 C-Atomen, insbesondere -OCH₃, -OC₂H₅, oder -OC₃H₇ oder ein Halogenatom, insbesondere -Cl oder eine Dialkylaminogruppe mit identischen oder unterschiedlichen Alkylresten mit 1 bis 6 C-Atomen;
R² und R³ unabhängig voneinander ein Kohlenwasserstoffrest mit 1 bis 10 C-Atomen, insbesondere -CH₃, -C₂H₅, oder -C₃H₇, oder einen Alkoxyrest mit 1 bis 10 C-Atomen, insbesondere -OCH₃, -OC₂H₅ oder -OC₃H₇, oder ein Halogenatom oder ein durch mindestens eine Oxa- oder Aminogruppe unterbrochener Alkylrest mit 4 bis 20 C-Atomen, wobei dieser Rest auch ein- oder mehrfach durch Halogen, Cyan, Nitro, Amino, Monoalkylamino, Dialkylamino, Hydroxy oder Aryl substituiert sein kann;
R⁴ eine Kohlenwasserstoffkette mit 1 bis 20 C-Atomen oder ein durch mindesten eine Oxa- oder Aminogruppe unterbrochener Alkylrest, wobei dieser Rest auch ein- oder mehrfach mit Halogen, Cyan, Nitro, Amino, Monoalkylamino, Dialkylamino, Alkoxy, Hydroxy, Aryl und/oder Epoxy substituiert sein kann, insbesondere 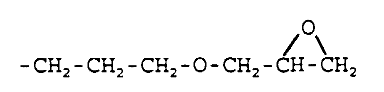 ist,
gefolgt von einem zweiten Schritt, wobei der im ersten Schritt modifizierte Träger umgesetzt wird mit einem Reagenz der allgemeinen Formel II:

X-R-Y (II),

wobei
X eine Amino-, Hydroxy-, Epoxy-Gruppe oder ein Halogenatom,
R eine Kohlenwasserstoffkette mit 2 bis 20 C-Atomen oder eine durch mindestens eine Oxa- oder Aminogruppe unterbrochenen Alkylrest, wobei dieser Rest auch ein- oder mehrfach mit Halogen, Cyan, Nitro, Amino, Monoalkylamino, Dialkylamino, Alkoxy, Hydroxy, Aryl und/oder Epoxy substituiert sein kann,
Y ein Kohlenwasserstoffrest mit Anionenaustauscher bildenenden funktionellen Gruppen mit 1 bis 10 C-Atomen, der ein- oder mehrfach mit Amino-, Monoalkylamino-, Dialkylamino-, Quartäralkylamino substituiert sein kann, ist.

Insbesondere kommen Trägermaterialien aus Silicagel in Betracht, auf deren Oberfläche Diethylaminoethyl (DEAE)-oder Diethylaminopropyl-Gruppen oder Dimethylaminoethyl (DMAE)-oder Dimethylaminopropyl-Gruppen entweder direkt oder über sogenannte Spacer angeordnet sind.

Im erfindungsgemäßen Verfahren wird insbesondere ein Anionenaustauscher der Formel 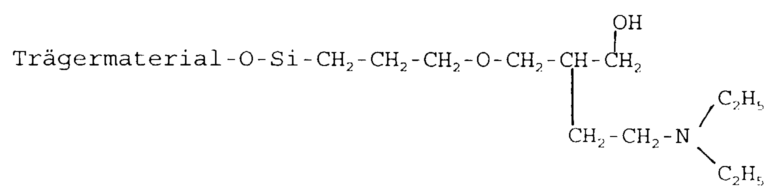 verwendet. Die Ethylgruppen des Amins können auch durch Methylgruppen ersetzt sein.

Die Nucleinsäuren können auch durch Anionenaustauscher-Materialien basierend auf Polystyrol/DvB, wie z. B. Poros 20 für Mitteldruckchromatographie, Poros® 50 HQ, der Firma BioPerseptive Cambridge, USA oder über DEAE-Sepharose®, Q-Sepharose®, DEAE-Sephadex® der Firma Pharmacia, Schweden; DEAE Spherodex® LS, DEAE Spherosil® der Firma Biosepra, Frankreich.

Auch Silica-Materialien, wie Kieselgur, Siloid®, Diatomeenerde, Glas, Silicagel, Aluminiumoxid, Titanoxid, Hydroxylapatit, in Anwesenheit von chaotropen Salzen, wie Natriumiodid, Guanidiniumhydrochlorid, und/oder Alkoholen sind zur Präparation der Nucleinsäuren erfindungsgemäß geeignet.

Bei der Präparation von Zellinhaltsstoffen, insbesondere von Nucleinsäuren, stellt sich oft das Problem, die aufgeschlossenen natürlichen Quellen, aus denen diese Inhaltsstoffe stammen von den gelösten Stoffen zu trennen. Die Abtrennung der Zellen oder Zelltrümmer erfolgt mittels Zentrifugation, wobei sich die größeren Zelltrümmer oder Zellen als Pellet im Zentrifugationsröhrchen abscheiden. Die Zellinhaltsstoffe finden sich dann im Überstand und können pipettiert werden. An sich einfachere Filtrationsverfahren konnten sich insbesondere bei der Präparation von Nucleinsäuren nicht durchsetzen, da die aufgeschlossenen Zellen oder deren Bruchstücke entweder durch die zu grobporige Filter durchlaufen und somit für Trübungen und Verunreinigungen im Filtrat sorgen oder bei Verwendung von Filtern mit entsprechenden engen Poren es jedoch zwangsläufig zur Verstopfung der Filter kommt, so daß eine sinnvolle Präparation der Zellinhaltsstoffe nicht mehr möglich ist.

Üblicherweise werden zur Entfernung von Zelltrümmern die Proben in 50 - 500 ml Gefäßen 5 - 60 min bei ca. 20.000 rpm (ca 30.000 x g) zentrifugiert.

Diese Zentrifugation ist zeitaufwendig und ist für größere Zell-Lysat-Volumina von 2 l und mehr kaum wirtschaftlich zu handhaben. Zwar gibt es Durchlaufzentrifugen, doch sind diese nur für sehr große Volumina > 1.000 l sinnvoll. Weiterhin ist dies aufwendig und kapitalintensiv.

Erfindungsgemäß wird eine einfache Entfernung von Zelltrümmern aus Zell-Lysaten von 1 l bis 1.000 l ermöglicht. Dabei werden die in der WO 93/11218 beschriebenen Filtrationsverfahren eingesetzt.

Erfindungsgemäß wird ebenfalls in besonders einfacher Weise bereits bei der Abtrennung von Zelltrümmern eine Endotoxinabreicherung oder -entfernung durchgeführt. Dies geschieht durch Anwendung des in PCT/EP 95/00392 vorgeschlagenen Verfahrens. Dabei wird der Zelltrümmer enthaltende Aufschluß über Filterschichten aus Glas, Silicagel, Diatomeenerde, Aluminiumoxiden, Titanoxiden, Zirkonoxiden, Hydroxylapatit oder anderen anorganischen Mineralien, wie z. B. Perlite® oder Filterschichten aus verwebten Vliesen, aus Glasfasern und Silicagel sowie Cellulose, Papier, gepreßtem Papier, verwebten oder verklebten Vliesen aus Polymeren, insbesondere Polypropylen, Polyamiden oder Polyester gegeben. Aluminiumoxid oder geschütteter Diatomeenerde oder verwebten oder verklebten Vliesen aus Glasfasern und Silicagel sowie Cellulose, Papier, gepreßtem Papier, Vliesen aus Papier gegeben. Die aus der Filterschicht austretende Fraktion wird aufgefangen und dann anschließend erfindungsgemäß weiterbehandelt.

Überraschenderweise zeigte sich, daß durch eine solche Filtration Endotoxine abgereichert werden. Es ist besonders bevorzugt, daß die Filterschicht bildenden Materialien Hydroxylgruppen tragen oder mit Hydroxylgruppen tragenden oder bildenden Orgnosilanen, wie z. B. 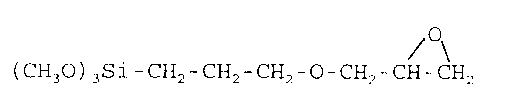 beschichtet bzw. modifiziert sind, insbesondere Diol-Silicagel, Diol-Diatomeenerde und/oder Diol-Perlite.

Insbesondere geschüttete Diatomeenerde hat sich zur Endotoxinabreicherung bzw. -entfernung bei der Probenvorbereitung bewährt.

Die nach dem erfindungsgemäßen Verfahren erhältliche Nucleinsäure ist auch für den Einsatz in der Gentherapie nach der "Anti-sense"- oder "Sense"-Strategie geeignet. Die "Anti-sense"-Strategie nutzt die Eigenschaft von zum Beispiel mRNA mit komplementären Nucleinsäuren Hybride zu formen. Die Hybride sind inaktiv. Die "Anti-sense"-Nucleinsäure inaktiviert also die mRNA. Die erfindungsgemäß erhaltene "Anti-sense"-RNA kann dabei von außen kontinuierlich dem zu therapierenden Individuum zugeführt werden, oder von entsprechend transformierten Zellen im Individuum selbst gebildet werden. Die "Sense"-Strategie führt zur Ergänzung oder Unterstützung von z.B. mRNA, die wichtige Funktionen erfüllt. Dabei wird die benötigte RNA dem zu therapierenden Individuum zugeführt. Die nach dem erfindungsgemäßen Verfahren erhältliche Nucleinsäure ist aufgrund ihrer hohen Reinheit auch geeignet, in sogenannten genetischen Vakzinierungsverfahren eingesetzt zu werden.

Das erfindungsgemäße Verfahren kann vorteilhaft ausgestaltet werden, indem die Entfernung des durch eine Elution der Nucleinsäuren unter Bedingungen hoher Ionenstärke notwendigen Salze durch Behandlung der hohen Salzkonzentrationen aufweisenden nucleinsäurehaltigen Fraktionen mit einem mineralischen Trägermaterial erfolgt. Diese Trägermaterialien bestehen im wesentlichen aus nicht modifizierten anorganischen Trägermaterialien z. B. Glas oder Glaspulver. An diese Oberflächen adsorbiert die Nucleinsäure in Gegenwart hoher Salzkonzentrationen. Danach kann die adsorbierte Nucleinsäure mit Lösungen geringer Ionenstärke oder entmineralisiertem Wasser desorbiert werden.

Es hat sich herausgestellt, daß die Verwendunq von isopropanolhaltigen Puffern anstelle von ethanolhaltigen Puffern vorteilhaft ist. Wie in PCT/WO 95/21177 vorgeschlagen, können durch die Verwendung von isopropanolhaltigen Puffern besonders gute Transfektionsraten der erfindungsgemäß hergestellten Nucleinsäuren erzielt werden.

Erfindungsgemäß beansprucht wird auch eine Zusammenstellung, die für die Durchführung des erfindungsgemäßen Verfahrens notwendigen Komponenten enthält. Dazu gehören insbesondere Reagenzien, auch in konzentrierter Form, zur fertigen Abmischung beim Anwender, Chromatographiematerial zur Trennung der Nucleinsäuren, wäßrige Lösungen (Puffer, gegebenenfalls auch in konzentrierter Form zu endgültigen Einstellung beim Anwender) sowie weitere Hilfsstoffe, wie Substanzen zur Entfernung von Endotoxinen, wie Diatomeenerde oder chromatographisches Material zur Entsalzung kochsalzeluierter Nucleinsäuren.

Das im erfindungsgemäßen Verfahren verwendbare Anionenaustauschermaterial ermöglicht DNA-Präparationen bis zum Kilogramm-Maßstab, insbesondere im Bereich von 10 mq bis 100 g DNA. Durch Untersuchung der DNA, die nach dem erfindungsgemäßen Verfahren isoliert wurde, mit Hilfe der HPLC-Analyse und Elektronenmikroskopie, zeigt, daß diese Präparationen frei von Proteinen (Endotoxinen), genomischer DNA sowie RNA sind. Die Erfindung wird anhand der folgenden Ausführungsbeispiele näher erläutert.

| | |
|---|---|
| Puffer P1 | 100 µg/ml RNAase A, 50 mM Tris/HCl, |
| (Resuspension buffer) | 10 mM EDTA, pH 8,0 |
| | |
| Puffer P2 | 200 mM NaOH, 1% SDS |
| (Lysis buffer) | |
| | |
| Puffer P3 | 3,0 M KAc, pH 5,5 |
| (Neutralisation buffer) | |
| | |
| Puffer QBT | 750 mM NaCl, 50 mM MOPS, |
| (Equilibration buffer) | 15% Alkohol*, pH 7,0, 0,15% Triton X-100 |
| | |
| Puffer QC | 1,0 M NaCl, 50 mM MOPS, |
| (Wash buffer) | 15% Alkohol, pH 7,0 |
| | |
| Puffer QN | 1,6 M NaCl, 50 mM MOPS |
| (Elution buffer) | 15% Alkohol, pH 8,5 |
| | |
| TE | 10 mM Tris/HCl, 1 mM EDTA, pH 8,0 |
| | |
| STE | 100 mM NaCl, 10 mM Tris/HCl, |
| | 1 mM EDTA, pH 8,0 |
| | |
| Endotoxin Removal Buffer | 750 mM NaCl, 10% Triton® X 114, |
| | 50 mM MOPS, pH 7,0 |

| | |
|---|---|
| * Als Alkohole werden bevorzugt Isopropanol oder Ethanol verwendet. | |

### Beispiel 1

Isolation von 50 mg pSVCFTR aus 10 1 E. coli Kultur für Aerosilation von CF-Patienten.

Das aus 10 l E.coli XL1 Blue Fermenterkultur resultierende Bakterienpellet wird in je 500 ml Puffer P1 und P2 resuspendiert. Mit 500 ml Puffer P3 wird das Gemisch für 30 min auf Eis inkubiert und anschließend für 15 min bei 20.000 x g zentrifugiert. Der Überstand wird zur Klärung über einen Faltenfilter filtriert. Das filtrierte Lysat wird mit 1/10 Vol. Endotoxin Removal Buffer (750 mM NaCl; 10% Triton X 114; 40 mM MOPS, pH 7,0) vermischt und für 30 min. auf Eis inkubiert. Das Lysat wird nun mittels einer Schlauchpumpe auf eine Chromatographiesäule 26 mm x 100 mm gefüllt mit einen QIAGEN Anionenaustauscher (70 - 100 µm Partikelgröße, 2,5 µmol DEAE/g Chromatographiematerial) adsorbiert. Anschließend wird die Chromatographiesäule mit 2.000 ml Puffer QC mit einer Flußrate von 15 ml/min gewaschen. Die an die Säule gebundene Plasmid DNA wird mit 280 ml Puffer QN, mit einer Flußrate von 3 ml/min eluiert. Das gewonnene Eluat wird mit 200 ml Isopropanol vermischt und für 30 min bei 20.000 x g zentrifugiert. Das resultierende Pellet wird in 40 ml TE Puffer resuspendiert. Die so gereinigte DNA wird mit Hilfe der Elektronenmikroskopie (EM), HPLC-Analyse, photometrische Messung, Agarosegel-Elektrophorese und Endotoxin-Test auf ihre Reinheit analysiert. Dabei resultiert diese Aufreinigungsmethode in hochreiner DNA mit keinen nachweisbaren Kontaminationen von RNA, genomischer DNA und Endotoxinen. Die so isolierte DNA wird mit einer Liposomenlösung vermischt und in Mengen von 10 µg CF-Patienten über Aerosilation verabreicht.

### Beispiel 2

Isolation von 10 mg pCMVlacZ mit Hilfe von QIAGEN tip-10.000 für Injektion von Plasmid-DNA in quergestreifte Muskel zur Behandlung von Muskeldystrophie.

5 l einer DH5alph/pCMVlacZ Übernachtkultur werden abzentrifugiert und das resultierende Pellet in 125 ml P1 resuspendiert, mit 125 ml Puffer P2 vermischt und für 5 min bei Raumtemperatur (RT) inkubiert. Anschließend wird 125 ml Puffer P 3 zugesetzt, gemischt und für 30 min bei 4°C inkubiert. Das Lysat wird über eine lose Schüttung von Diatomeenerde wie in PCT/EP 95/00392 beschriebene Filtrationssäule gegeben und anschließend wie in Beispiel 1 mit 1/10 Vol. Endotoxin Removal Puffer versetzt und für 30 min. auf Eis inkubiert. Das Gemisch wird nun mit 270 ml Isopropanol versetzt und für 30 min bei 20.000 x g abzentrifugiert. Das resultierende Pellet wird für 10 min bei RT getrocknet und in 5 ml Wasser resuspendiert. Die resuspendierte DNA wird mit 25 ml QC Puffer versetzt. Dieses Gemisch wird auf eine mit 75 ml QBT Puffer äquilibrierte DEAE-Silicagel-Säule (26 mm x 50 mm, 70 - 100 µm, 2,0 µmol/g) gegeben. Anschließend wird die Säule mit 600 ml QC Puffer gewaschen und die DNA dann mit 75 ml QF Puffer eluiert. Das Eluat wird mit 52,5 ml Isopropanol vermischt und für 30 min bei 20.000 x g zentrifugiert. Das DNA Pellet wird 10 min bei RT getrocknet und in 1 ml PBS resuspendiert. Die DNA-Lösung kann nur zur direkten Muskelinjektion verwendet werden.

### Beispiel 3

Isolation von 100 mg pXYHBV aus einer 20 1 E. coli Kultur für die Verwendung als "Genetische Hepatitis Vakzine".

Das aus einem 20 l Fermentationslauf resultierende Bakterienpellet wird in 1.000 ml Puffer P1 resuspendiert, mit 1.000 Puffer P2 versetzt und für 5 min bei RT inkubiert. Nach dem Zusatz von 1.000 ml Puffer P3 wird das Gemisch für 30 min bei 4°C inkubiert und anschließend bei 20.000 x g zentrifugiert. Der Überstand wird über ein Glasfaserfilter gegeben und das klare Lysat wird mit 2.250 ml Isopropanol vermischt und für 30 min bei 20.000 x g zentrifugiert. Das resultierende Pellet wird in 10 ml Wasser resuspendiert und mit 90 ml QC Puffer versetzt. Dieses Gemisch wird über eine Schlauchpumpe auf eine Chromatographiesäule nach Beispiel 1 mit einer Flußrate von 2 ml/min gepumpt. Die Säule wird mit einer Flußrate von 15 ml/min gewaschen und die DNA im Anschluß mit 350 ml QF Puffer mit einer Flußrate von 3 ml/min eluiert.

### Beispiel 4

### Entfernen von Endotoxin aus DNA Präparationen

Die präparierte DNA wird mit Triton® X 114 auf eine Endkonzentration von 0,1 - 1% eingestellt. Anschließend wird die DNA/Triton Lösung für 30 min bei 4 - 7°C auf einem "Roller" inkubiert. Die Lösung wird auf Raumtemperatur erwärmt und für 30 min bei 20.000 x g zentrifugiert oder filtriert. Der Überstand wird mit 0,7 Vol. Isopropanol versetzt und gefällt. Das resultierende Pellet wird getrocknet und in TE resuspendiert. Die so behandelte DNA ist frei von Endotoxin.

### Beispiel 5

### Plasmidpräparation

Eine 150 ml HB 101 E.coli Kultur mit pUC 18 Plasmid-DNA in LB-Medium wird 5 Minuten bei 3.000 g zentrifugiert, um die Zellen zu pelletieren. Das Zellpellet wird in 20 ml 50 ml Tris-Hcl, 10 mM EDTA, pH 8,0, 100 µg/ml RNAse A resuspendiert Zur Zell-Lyse werden 20 ml 0,2 M NaOH, 1% SDS zur Zellsuspension gegeben, vorsichtig gemischt und 5 Minuten bei Raumtemperatur stehengelassen. Danach wird 20 ml 3 M K-Acetat, 2 M Essigsäure zur Neutralisation zugegeben, gemischt und 15 Minuten auf Eis inkubiert und das Zell-Lysat 2.000 Pa bis 80.000 Pa mit (20 mbar - 800 mbar) Differenzdruck durch die erfindungsgemäße Filtervorrichtung gesaugt. Alternativ kann die Probe mit einem Kolbenstempel oder Überdruck durch die Filtrationsschichten gedrückt werden. Nach der Filtration wird die Filtrationsvorrichtung abgenommen und der Filterkuchen mit den Zellbruchstücken, den denaturierten Proteinen und dem ausgefallenen SDS verworfen. Das filtrierte Lysat wird mit 1/10 Vol. Endotoxin Removal Buffer (750 mM NaCl; 10% Triton® X 114; 40 mM MOPS, pH 7,0) vermischt und für 30 min. auf Eis inkubiert. Das Filtrat wird vollständig durch die Anionenaustauschersäule gesaugt oder gedrückt, um eine Adsorption der DNA zu erreichen. Die Extraktionssäule wird anschließend zweimal mit 100 ml 1 M NaCl, 15% Ethanol, 50 mM MOPS, pH 7,0 gewaschen, um RNA und Proteine zu entfernen. Die DNA wird mit 100 ml 1,6 M NaCl, 15% Ethanol, 50 mM MOPS, pH 7,0 eluiert. Die eluierte DNA wird zur Entsalzung und zur Konzentrierung mit Alkohol gefällt und das Alkoholpellet durch eine Zentrifugation pelletiert.

Alternativ kann das Alkoholpräzipitat der Nucleinsäure auch durch eine Filtration gewonnen werden. Dies bietet Vorteile, wenn großen Mengen an DNA präpariert werden müssen und die zu handhabenden Volumina größer als beispielsweise 1 l sind.

### Beispiel 6

Ein DNA-Template wird über eine in vitro Reaktion in RNA umgeschrieben. Der Reaktionsansatz wird auf 750 mM NaCl eingestellt und über eine QIAGEN® Anionenaustauscher-Säule aufgereinigt. Die gereinigte RNA wird anschließend zur in vitro oder in vivo Gentherapie verwendet.

### Beispiel 7

Aufreinigung von 40 mg pBR322 mit Hilfe von DEAE Q-Sepharose® (Firma Pharmacia)

Die Biomasse aus einer 401 Fermenter Kultur von pBR322 wurde mit jeweils 101 Puffern P1, P2 und P3 mittels alkalischer Lyse aufgeschlossen. Abschließend wurde das Lysat über eine Filtervorrichtung, bestehend aus einer losen Schüttung versetzt und anschließend für 30 min bei 4°C inkubiert. Die DNA wird nun durch Zugabe von 0,7 Vol. Isopropanol präzipitiert und in 20 ml 10 mM Tris-HCl, pH 8,5, 1 mM EDTA, 50mM NaCl resuspendiert. Die resuspendierte DNA-Lösung wird auf eine DEAE Q-Sepharose-Säule mit 200 ml Bettvolumen geladen. Die DNA wird mit einem Gradiente von 1 mM NaCl/ml eluiert, wobei die Puffer folgende Konzentrationen aufweisen:

Buffer A 10 mM Tris HCl, 1mM EDTA, 0,75 M NaCl, pH 8,0; Buffer B 10 mM Tris HCl, 1mM EDTA, 0,85 M NaCl.

Die Flußrate beträgt dabei 0,5 ml/min. Die DNA wird anschließend mit Ethanol gefällt und in PBS Buffer in einer Konzentration von 1 µm/µl resuspendiert.

## Patentansprüche

1. Verfahren zur Isolierung und Reinigung von Nucleinsäuren und/oder Oligonucleotiden Für den Einsatz in der Gentherapie, wobei die Nucleinsäuren und/oder Oligonucleotide aus einer im wesentlichen biologischen Quelle isoliert oder gereinigt werden, dadurch gekennzeichnet, daß
- a) die im wesentlichen biologischen Quellen aufgeschlossen werden, die erhaltenen Fraktionen gegebenenfalls vom Rest der biologischen Quellen befreit oder abgereichert werden durch an sich bekannte mechanische Verfahren, wie Zentrifugieren, Filtrieren,
- b) die so behandelten Fraktionen mit nicht ionischen Detergenzien wie Triton® X 114 oder affinitätschromatographischen Trägern, wie Nickel-NTA, -IDA, Polymyxin, DNA ETOX® oder mit anorganischem Chromatographie-Material zur Entfernung von Endotoxinen behandelt werden,
- c) sich eine Isolierung der Nucleinsäuren und/oder Oligonucleotide an einem Anionenaustauscher anschließt, der so beschaffen ist, daß DNA erst bei einer mindestens ca. 100 mM höheren Natriumchloridlösung entsprechenden Ionenstärke vom Anionenaustauscher zu desorbieren beginnt als derjenigen Ionenstärke entspricht, bei der RNA vom Anionenaustauschermaterial zu desorbieren beginnt.

2. Verfahren nach Anspruch 1, dadurch gekennzeichnet, daß als Trägermaterial des anorganischen Chromatographie-Matcrials mit Anionenaustauschergruppen modifizierte poröse oder nicht poröse anorganische und/oder organische Trägermaterialien verwendet werden.

3. Verfahren nach einem der Ansprüche 1 und/oder 2, dadurch gekennzeichnet, daß als anorganische Trägermaterialien Silicagel, Diatomeenerde, Glas, Aluminiumoxide, Titanoxide, Zirkonoxide, Hydroxylapatit und als organische Trägermaterialien Dextran, Agarose, Acrylamid, Polystyrolharze oder Copolymere der genannten Materialien verwendet werden.

4. Verfahren nach mindestens einem der Ansprüche 1 - 3, wobei das modifizierte Trägermaterial erhältlich ist durch Umsetzung eines der in Anspruch 3 genannten Trägermaterialien in einem ersten Schritt mit einem Silanisierungsreagenz der allgemeinen Formel I
R¹R²R³Si R⁴ (I),
wobei
R¹ ein Alkoxyrest mit 1 bis 10 C-Atomen, inebesondere -OCH₃, -OC₂H₅, oder -OC₃H₇ oder ein Halogenatom, insbesondere -Cl oder eine Dialkylaminogruppe mit identischen oder unterschiedlichen Alkylresten mit 1 bis 6 C-Atomen;
R² und R³ unabhängig voneinander ein Kohlenwasserstoffrest mit 1 bis 10 C-Atomen, insbesondere -CH₃, -C₂H₅, oder -C₃H₇, oder ein Alkoxyrest mit 1 bis 10 C-Atomen, insbesondere -OCH₃, -OC₂H₅, oder -OC₃H₇, oder ein Halogenatom oder ein durch mindestens eine Oxa- oder Aminogruppe unterbrochener Alkylrest mit 4 bis 20 C-Atomen, wobei dieser Rest auch ein- oder mehrfach durch Halogen, Cyan, Nitro, Amino, Monoalkylamino, Dialkylamino, Hydroxy oder Aryl substituiert sein kann;
R⁴ eine Kohlenwasserstoffkette mit 1 bis 20 C-Atomen oder ein durch mindesten eine Oxa- oder Aminogruppe unterbrochener Alkylrest, wobei dieser Rest auch ein- oder mehrfach mit Halogen, Cyan, Nitro, Amino, Monoalkylamino, Dialkylamino, Alkoxy, Hydroxy, Aryl und/oder Epoxy substituiert sein kann, insbesondere 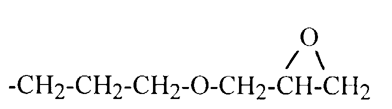 ist,
gefolgt von einem zweiten Schritt, wobei der im ersten Schritt modifizierte Träger umgesetzt wird mit einem Reagenz der allgemeinen Formel II:
X-R-Y (II),
wobei
X eine Amino-, Hydroxy-, Epoxy-Gruppe oder ein Halogenatom,
R eine Kohlenwasserstoffkette mit 2 bis 20 C-Atomen oder eine durch mindestens eine Oxa- oder Aminogruppe unterbrochenen Alkylrest, wobei dieser Rest auch ein- oder mehrfach mit Halogen, Cyan, Nitro, Amino, Monoalkylamino, Dialkylamino, Alkoxy, Hydroxy, Aryl und/oder Epoxy substituiert sein kann,
Y ein Kohlenwasserstoffrest mit Anionenaustauscher bildenden funktionellen Gruppen mit 1 bis 10 C-Atomen, der ein- oder mehrfach mit Amino-, Monoalkylamino, Dialkylamino-, Quartäralkylamino substituiert sein kann, ist.

5. Verfahren nach mindestens einem der Ansprüche 1 bis 4, wobei auf der Trägeroberfläche Diethylaminoethyl (DEAE)-Gruppen oder Dimethylaminoethyl (DMAE)-Gruppen entweder direkt oder über sogenannte Spacer angeordnet sind.

6. Verfahren nach mindestens einem der Ansprüche 1 bis 5, wobei als Nucleinsäuren DNA wie Plasmide, Cosmidc, aus Viren isolierte DNA auch in enzymatisch oder chemisch modifizierter Form und/oder RNA jeglicher Form und Herkunft oder Ribozyme isoliert werden.

7. Verfahren nach einem der Ansprüche 1 bis 6, wobei eine Entfernung des durch eine Elution der Nucleinsäuren unter Bedingungen hoher Ionenstärke notwendigen Salze durch Behandlung der hohe Salzkonzentrationen aufweisenden nucleinsäurehaltigen Fraktionen mit einem mineralischen Trägermaterial, wie im wesentlichen aus Glas bestehenden, unter Adsorption der Nucleinsäure an die Oberfläche dieser mineralischen Träger und anschließender Desorption der Nucleinsäuren mit Wasser oder Pufferlösungen geringer Ionenstärke.

8. Verfahren nach einem der Ansprüche 1 bis 6, wobei Zelltrümmer des Zell-Lysates durch Filtration abgetrennt werden, insbesondere unter Vcrwendung eines Filters, dessen Porengröße in Fließrichtung der zu filtrierenden Probe abnimmt und/oder einem Filter, der eine Filterschicht aus Glas, Silicagel, Aluminiumoxid oder geschütteter Diatomeenerde oder verwebte oder verklebte Vliese aus Glasfasern und Silicagel sowie Cellulose, Papier, gepreßtem Papier, Vliesen aus Papier besteht.

9. Verfahren nach mindestens einem der Ansprüche 1 bis 8, wobei eine Vorreinigung einer nucleinsäurehaltigen Probe eines Zell-Lysats an einer Schicht aus unmodifizierter Diatomeenerde erfolgt.

10. Verwendung von Anionenaustauschermaterialien für die Trennung, Reinigung und Isolierung von Nucleinsäuren zur Herstellung eines nucleinsäurchaltigen Mittels für die Gentherapie in einem Verfahren gemäß einem der Ansprüche 1 bis 9.

11. Verwendung nach Anspruch 10 für die in vivo- und ex vivo-Gentherapie.

12. Verwendung gemäß einem der Ansprüche 10 oder 11 zur Herstellung eines Mittels zur Behandlung von genetisch bedingten Erkrankungen wie cystische Fibrose, Muskeldystrophie.

13. Verwendung von einem Anionenaustauschermaterial für die Reinigung von Oligonucleotiden für die in vivo-/ex vivo-Gentherapie über die Antisense-, Sense-Strategie in einem Verfahren gemäß Anspruch 1 bis 9.

14. Verwendung von Anionenaustauschermaterial für die Aufreinigung von Viruspartikeln, auch intakten Viruspartikeln für die in vivo-/ex vivo-Gentherapie in einem Verfahren gemäß Anspruch 1 bis 9.

15. Verwendung von Isopropanol als Reagenz zur Aufreinigung von Nucleinsäuren in einem Verfahren gemäß einem der Ansprüche 1 bis 9.

16. Kit, enthaltend Reagenzien, auch in konzentrierter Form, zur fertigen Abmischung beim Anwender, Chromatographie-Material zur Trennung der Nucleinsäuren, die folgenden wäßrigen Lösungen (Puffer, gegebenenfalls auch in konzentrierter Form zur endgültigen Einstellung beim Anwender):
Puffer P2: 200 mM NaOH, 1% SDS,
Puffer QN: 1,6 M NaCl, 50 mM MOPS, 15% Alkohol, pH 8,5 sowie
Endotoxin Removal Buffer: 750 mM NaCl, 10% Triton® X 114, 50 mM MOPS, pH 7,0,
weitere Hilfsstoffe sowie Substanzen zur Entfernung von Endotoxinen für die Durchführung des Verfahrens nach einem der Ansprüche 1 bis 9.

## Claims

1. A process for the isolation and purification of nucleic acids and/or oligonucleotides for use in gene therapy wherein said nucleic acids and/or oligonucleotides are isolated or purified from an essentially biological source, characterized in that
- a) said essentially biological sources are lysed, the fractions obtained are optionally freed or depleted from the remainder of said biological sources by per se known mechanical methods, such as centrifugation, filtration;
- b) the fractions thus treated are treated with nonionic detergents, such as Triton® X114, or affinity chromatographic supports, such as nickel-NTA, nickel-IDA, polymyxin, DNA-ETOX®, or with inorganic chromatographic material for the removal of endotoxins;
- c) followed by isolation of said nucleic acids and/or oligonucleotides on an anion exchanger which is designed such that DNA begins to desorb from the anion exchanger only at an ionic strength corresponding to a sodium chloride solution of a concentration higher by at least 100 mM than one corresponding to the ionic strength at which RNA begins to desorb from the anion exchanger material.

2. The process according to claim 1, characterized in that porous or non-porous inorganic and/or organic support materials modified with anion exchanging groups are used as the support material of said inorganic chromatographic material.

3. The process according to any of claims 1 and/or 2, characterized in that silica gel, diatomaceous earth, glass, aluminium oxides, titanium oxides, zirconium oxides, hydroxyapatite are used as inorganic support materials, and dextrane, agarose, acrylic amide, polystyrene resins, or copolymers of the materials mentioned are used as organic support materials.

4. The process according to at least one of claims 1 to 3, wherein said modified support material is obtained by reacting one of the support materials mentioned in claim 3 in a first step with a silanizing reagent of general formula I,
R¹R²R³SiR⁴ (I)
wherein
R¹ is an alkoxy residue of from 1 to 10 carbon atoms, especially -OCH₃, -OC₂H₅ or -OC₃H₇, or a halogen atom, especially -Cl, or a dialkylamino group with identical or different alkyl residues of from 1 to 6 carbon atoms;
R² and R³ are independently a hydrocarbon residue of from 1 to 10 carbon atoms, especially -CH₃, -C₂H₅ or -C₃H₇, or an alkoxy residue of from 1 to 10 carbon atoms, especially -OCH₃, -OC₂H₅ or -OC₃H₇, or a halogen atom, or an alkyl residue of from 4 to 20 carbon atoms which is interrupted by at least one oxa or amino group wherein said residue may also be substituted with one or more of halogen, cyano, nitro, amino, monoalkylamino, dialkylamino, hydroxy or aryl;
R⁴ is a hydrocarbon chain of from 1 to 20 carbon atoms, or an alkyl residue which is interrupted by at least one oxa or amino group wherein said residue may also be substituted with one or more of halogen, cyano, nitro, amino, monoalkylamino, dialkylamino, alkoxy, hydroxy, aryl, and/or epoxy, especially 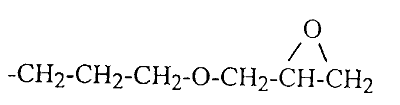 followed by a second step wherein the support which has been modified in the first step is reacted with a reagent of general formula II:
X-R-Y (II)
wherein
X is an amino, hydroxy, epoxy group or a halogen atom;
R is a hydrocarbon chain of from 2 to 20 carbon atoms, or an alkyl residue which is interrupted by at least one oxa or amino group wherein said residue may also be substituted with one or more of halogen, cyano, nitro, amino, monoalkylamino, dialkylamino, alkoxy, hydroxy, aryl, and/or epoxy;
Y is a hydrocarbon residue having functional groups which form an anion exchange material and having from 1 to 10 carbon atoms which may be substituted with one or more of amino, monoalkylamino, dialkylamino, quarternary alkylamino.

5. The process according to at least one of claims 1 to 4, wherein diethylaminoethyl (DEAE) groups or dimethylaminoethyl (DMAE) groups are provided on the surface of the support either directly or through so-called spacers.

6. The process according to at least one of claims 1 to 5, wherein DNA, such as plasmids, cosmids, DNA isolated from viruses, also in enzymatically or chemically modified form, and/or RNA in any form and of any origin, or ribozymes are isolated as said nucleic acids.

7. The process according to any of claims 1 to 6, wherein the salts, which are necessary for eluting the nucleic acids under conditions of high ionic strength, are removed by treating with a mineral support material, such as one essentially consisting of glass, the fractions containing nucleic acids and having the high salt concentrations, wherein the nucleic acid adsorbs to the surface of said mineral supports, and subsequent desorption of the nucleic acids with water or buffer solutions of low ionic strength.

8. The process according to any of claims 1 to 6, wherein cell debris of the cell lysate are separated off by filtration, in particular using a filter the pore size of which decreases in the direction of flow of the sample to be filtrated and/or a filter having a filter layer of glass, silica gel, alumina or packed diatomaceous earth or interlaced or bonded non-wovens made of fiber glass and silica gel as well as cellulose, paper, pressed paper, non-wovens made of paper.

9. The process according to at least one of claims 1 to 8, wherein a preliminary purification of a sample of a cell lysate containing nucleic acids is performed on a layer of unmodified diatomaceous earth.

10. Use of anion exchange materials for the separation, purification and isolation of nucleic acids for the preparation of an agent containing nucleic acids for gene therapy in a process according to any of claims 1 to 9.

11. The use according to claim 10 for in vivo and ex vivo gene therapy.

12. The use according to any of claims 10 or 11 for the preparation of an agent for the treatment of genetically caused diseases, such as cystic fibrosis, muscular dystrophy.

13. Use of an anion exchange material for the purification of oligonucleotides for in vivo/ex vivo gene therapy by the antisense/sense strategy in a process according to claims 1 to 9.

14. Use of an anion exchange materials for the purification of virus particles, also intact virus particles, for in vivo/ex vivo gene therapy in a process according to claims 1 to 9.

15. Use of isopropanol as a reagent for the purification of nucleic acids in a process according to any of claims 1 to 9.

16. A kit containing reagents, also in concentrated form for final mixing by the user, chromatographic materials for the separation of the nucleic acids, the following aqueous solutions (buffers, optionally also in concentrated form for final adjusting by the user):
buffer P2: 200 mM NaOH, 1% SDS;
buffer QN: 1.6 M NaCl, 50 mM MOPS, 15% alcohol, pH 8.5; and
endotoxin removal buffer: 750 mM NaCl, 10% Triton® X114, 50 mM MOPS, pH 7.0;
further auxiliaries, and substances for the removal of endotoxins, for performing the process according to any of claims 1 to 9.

## Revendications

1. Procédé pour isoler et purifier des acides nucléiques et/ou des oligonucléotides pour utilisation en thérapie génique, où les acides nucléiques et/ou oligonucléotides sont isolés ou purifiés à partir d'une source essentiellement biologique, caractérisé en ce que
a) les sources essentiellement biologiques sont attaquées, les fractions obtenues sont éventuellement débarrassées du reste des sources biologiques ou appauvries par des procédés mécaniques connus en soi, tels qu'une centrifugation, une filtration,
b) les fractions ainsi traitées sont traitées avec des détergents non ioniques tels que le Triton® X 114 ou des supports pour chromatographie d'affinité, tels que le nickel-NTA, le nickel-IDA, la polymyxine, le DNA ETOX®, ou avec un matériau minéral pour chromatographie, pour éliminer les endotoxines,
c) puis on procède à un isolement des acides nucléiques et/ou des oligonucléotides sur un échangeur d'anions, qui est tel que l'ADN ne commence à se désorber de l'échangeur d'anions que pour une force ionique correspondant à une solution de chlorure de sodium d'au moins environ 100 mM plus grande que la force ionique pour laquelle l'ARN commence à se désorber du matériau échangeur d'anions.

2. Procédé selon la revendication 1, caractérisé en ce qu'on utilise en tant que matériau support du matériau minéral pour chromatographie des matériaux supports inorganiques et/ou organiques, poreux ou non poreux, modifiés par des groupes échangeurs d'anions.

3. Procédé selon l'une des revendications 1 et/ou 2, caractérisé en ce qu'on utilise en tant que matériaux supports minéraux du gel de silice, de la terre de diatomées, du verre, des oxydes d'aluminium, des oxydes de titane, des oxydes de zirconium, de l'hydroxyapatite et, en tant que matériaux supports organiques, du dextrane, de l'agarose, de l'acrylamide, des résines de polystyrène ou des copolymères des matériaux mentionnés.

4. Procédé selon au moins l'une des revendications 1 à 3, dans lequel le matériau support modifié peut être obtenu par réaction de l'un des matériaux supports mentionnés dans la revendication 3, dans une première étape avec un réactif de silanisation de formule générale I
R¹R²R³SiR⁴ (I)
dans laquelle
R¹ est un radical alcoxy ayant de 1 à 10 atomes de carbone, en particulier -OCH₃, -OC₂H₅ ou -OC₃H₇, ou un atome d'halogène, en particulier -Cl, ou un groupe dialkylamino comportant des radicaux alkyles identiques ou différents ayant de 1 à 6 atomes de carbone ;
R² et R³ représentent chacun indépendamment de l'autre un radical hydrocarboné ayant de 1 à 10 atomes de carbone, en particulier -CH₃, -C₂H₅ ou -C₃H₇, ou un radical alcoxy ayant de 1 à 10 atomes de carbone, en particulier -OCH₃, -OC₂H₅ ou -OC₃H₇, ou un atome d'halogène, ou un radical alkyle ayant de 4 à 20 atomes de carbone, interrompu par au moins un groupe oxa ou amino, ce radical pouvant aussi être une ou plusieurs fois substitué par des substituants halogéno, cyano, nitro, amino, monoalkylamino, dialkylamino, hydroxy ou aryle ;
R⁴ est une chaîne hydrocarbonée ayant de 1 à 20 atomes de carbone ou un radical alkyle interrompu par au moins un groupe oxa ou amino, ce radical pouvant aussi être substitué une ou plusieurs fois par des substituants halogéno, cyano, nitro, amino, monoalkylamino, dialkylamino, alcoxy, hydroxy, aryle et/ou époxy, en particulier 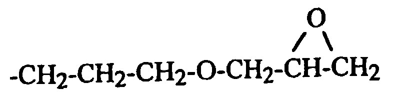
puis par une deuxième étape, dans laquelle le support modifié dans la première étape est mis à réagir avec un réactif de formule générale II
X-R-Y (II)
dans laquelle
X est un groupe amino, hydroxy, époxy ou un atome d'halogène,
R est une chaîne hydrocarbonée ayant de 2 à 20 atomes de carbone ou un radical alkyle interrompu par au moins un groupe oxa ou amino, ce radical pouvant aussi être une ou plusieurs fois substitué par des substituants halogéno, cyano, nitro, amino, monoalkylamino, dialkylamino, alcoxy, hydroxy, aryle et/ou époxy,
Y est un radical hydrocarboné comportant des groupes fonctionnels formant des échangeurs d'anions, ayant de 1 à 10 atomes de carbone, et qui peut être une ou plusieurs fois substitué par des substituants amino, monoalkylamino, dialkylamino, alkylamino quaternaires.

5. Procédé selon au moins l'une des revendications 1 à 4, dans lequel des groupes diéthylaminoéthyle (DEAE) ou des groupes diméthylaminoéthyle (DMAE) sont disposés sur la surface du support, soit directement, soit par l'intermédiaire de ce que l'on appelle des espaceurs.

6. Procédé selon au moins l'une des revendications 1 à 5, dans lequel, en tant qu'acides nucléiques, on isole des ADN tels que des plasmides, des cosmides, des ADN isolés de virus, y compris sous une forme ayant subi une modification enzymatique ou chimique et/ou des ARN sous toutes formes et de toutes origines, ou des ribozymes.

7. Procédé selon l'une des revendications 1 à 6, dans lequel on procède à une élimination des sels, nécessaires du fait d'une élution des acides nucléiques dans les conditions d'une grande force ionique, par traitement des fractions contenant des acides nucléiques, présentant de fortes concentrations de sels, avec un matériau support minéral, essentiellement en verre, par adsorption de l'acide nucléique par la surface de ces supports minéraux, puis désorption des acides nucléiques avec de l'eau ou avec des solutions tampons ayant une faible force ionique.

8. Procédé selon l'une des revendications 1 à 6, dans lequel les débris cellulaires du lysat cellulaire sont séparés par filtration, en particulier par utilisation d'un filtre dont le diamètre des pores diminue dans la direction d'écoulement de l'échantillon à filtrer, et/ou d'un filtre constitué d'une couche filtrante en verre, gel de silice, oxyde d'aluminium ou terre de diatomées en vrac, ou encore est constituée de nappes tissées ou collées de fibres de verre et de gel de silice, ainsi que de cellulose, de papier, de papier comprimé, de nappes de papier.

9. Procédé selon au moins l'une des revendications 1 à 8, dans lequel on procède à une purification préalable d'un échantillon contenant des acides nucléiques d'un lysat cellulaire sur une couche en terre de diatomées non modifiée.

10. Utilisation de matériaux échangeurs d'anions pour la séparation, la purification et l'isolement d'acides nucléiques, pour préparer un produit contenant des acides nucléiques, pour la thérapie génique, dans le cadre d'un procédé selon l'une des revendications 1 à 9.

11. Utilisation selon la revendication 10, pour la thérapie génique in vivo et ex vivo.

12. Utilisation selon l'une des revendications 10 ou 11, pour préparer un produit destiné au traitement de maladies d'origine génétique, telles que la mucoviscidose, la myopathie primitive progressive.

13. Utilisation d'un matériau échangeur d'anions pour purifier des oligonucléotides pour la thérapie génique in vivo/ex vivo, par l'intermédiaire d'une stratégie antisens-sens dans le cadre d'un procédé selon les revendications 1 à 9.

14. Utilisation d'un matériau échangeur d'anions pour purifier des particules virales, et même des particules virales intactes, pour la thérapie génique in vivo/ex vivo dans le cadre d'un procédé selon les revendications 1 à 9.

15. Utilisation d'isopropanol en tant que réactif pour purifier des acides nucléiques dans le cadre d'un procédé selon l'une des revendications 1 à 9.

16. Trousse contenant des réactifs, y compris sous forme concentrée, pour mélange extemporané par l'utilisateur, un matériau pour chromatographie destiné à séparer les acides nucléiques, les solutions aqueuses suivantes (tampons, éventuellement aussi sous forme concentrée pour ajustement définitif par l'utilisateur) :
tampon P2 : NaOH 200 mM, 1 % SDS,
tampon QN : NaCl 1,6 M, MOPS 50 mM, 15 % alcool, pH 8,5, et aussi
tampon d'élimination des endotoxines : NaCl 750 mM, 10 % Triton® X 114, MOPS 50 mM, pH 7,0,
d'autres adjuvants, ainsi que des substances pour éliminer les endotoxines, pour mettre en oeuvre le procédé selon l'une des revendications 1 à 9.
